**Europäisches Patentamt**

**European Patent Office**

**Office européen des brevets**

(11) Veröffentlichungsnummer: **0 460 212 A1**

(12) **EUROPÄISCHE PATENTANMELDUNG**
veröffentlicht nach Art. 158 Abs. 3
**EPÜ**

(21) Anmeldenummer: **90901648.7**

(51) Int. Cl.⁵: **A61N 5/06**

(22) Anmeldetag: **15.09.89**

(86) Internationale Anmeldenummer:
**PCT/SU89/00245**

(87) Internationale Veröffentlichungsnummer:
**WO 91/04072 (04.04.91 91/08)**

(43) Veröffentlichungstag der Anmeldung:
**11.12.91 Patentblatt 91/50**

(84) Benannte Vertragsstaaten:
**AT CH DE FR GB IT LI SE**

(71) Anmelder: **DONETSKY GOSUDARSTVENNY
MEDITSINSKY INSTITUT IMENI M.GORKOGO
Pr. Iliicha, 16
Donetsk, 340098(SU)**

Anmelder: **VOROSHILOVGRADSKY
MEDITSINSKY INSTITUT
Kvartal 50-letia Oborony Luganska
Voroshilovgrad, 348045(SU)**

(72) Erfinder: **CHUPRIKOV, Anatoly Pavlovich
16 linia, 23a-33
Voroshilovgrad, 348016(SU)**

Erfinder: **MARTSENKOVSKY, Igor Anatolievich
Leninsky pr., 14-48
Donetsk, 340045(SU)**
Erfinder: **BUSURIN, Mikhail Jurievich
ul. Universitetskaya, 59-5
Donetsk, 340050(SU)**
Erfinder: **LEKAKH, Viktor Aronovich
pr. Grinkevicha, 7-44
Donetsk, 340055(SU)**
Erfinder: **SAVITSKY, Stanislav Jurievich
bulvar Shevchenko, 18-43
Donetsk, 340100(SU)**

(74) Vertreter: **Lehn, Werner, Dipl.-Ing. et al
Hoffmann, Eitle & Partner Patentanwälte
Arabellastrasse 4
W-8000 München 81(DE)**

(54) **ANORDNUNG ZUR KORREKTUR DES GEMÜTSZUSTANDES EINER PERSON.**

(57) Die Erfindung bezieht sich auf das Gebiet Medizin.

Die Einrichtung zur Korrektion des Gefühlzustandes einer Person enthält eine Lichtquelle (1) mit ihrer optischen Strahlung (15), mindestens ein zwischen der Lichtquelle (1) und dem Auge (7) angeordnetes Lichtfilter (2), das zwei Filterhälften (3, 4) vorsieht, die zur Formgebung von zwei Lichtströme (10, 11) mit unterschiedlichen Wellenlängen aus der erwähnten optischen Strahlung (14) die Trenngrenze (5) trennt, welche einen gleichzeitigen Eingriff mit einem Lichtstrom (10) an der linken und mit dem anderen Lichtstrom (11) an der rechten Netzhauthälfte (16) bzw. 16' sdes Auges 7 bis zum Verschwinden der persönlichen Wahrnehmung der Farbendifferenz ermöglicht.

EP 0 460 212 A1

FIG.1

Technisches Gebiet

Die vorliegende Erfindung bezieht sich auf das Gebiet Medizin, insbesondere auf Physiotherapie, und zwar auf Einrichtungen zur Korrektion des Gefühlzustandes einer Person.

Zugrundeliegender Stand der Technik

Zum besseren Verständis der Erfindung werden nachstehend die Erläuterungen einzelner Begriffe angeführt.

Unter einer Korrektion des Gefühlzustandes sind die auf Veränderung des Gefühlzustandes einer Person ausgerichteten Handlungen, insbesondere alle Heilungshandlungen zu verstehen.

Unter der Sagittalebene eines Auges wird hierbei eine Ebene verstanden, in welcher die das Auge in Längsrichtung in rechte und linke Aussenhälfte zerteilende Ebene liegt.

Zur Korrektion des Gefühlzustandes einer Person werden zur Zeit die chemischtherapeutischen Präparate ausgenutzt, die hauptsächlich unter Berücksichtigung der Modalität und Intensität der Affekterscheinungen und wesentlich weniger mit Rücksicht auf deren Struktur und neurobiologische Organisation verordnet werden (G.Ya.Avrutsky und andere "Leitfaden für Ärzte", 1981, Medizin, Moskau, S. 496).

Allgemein bekannt wird eine Pathomorfose des klinischen Verlaufs der Affektstörungen vermerkt, die neben dem Einfluss unterschiedlicher sozialer und kultureller Faktoren auch die Folge einer weitgehenden Anwendung der grossen Anzahl von Tranquilizern und Antidepressanten sowohl in der polyklinischen Praxis als auch zwecks Selbstheilung ist (G.Ya.Avrustky und andere "Pharmakotherapie der psychischen Erkrankungen", 1974, Medizin, Moskau, S. 154). Die erwähnten Autoren sind auch der Meinung, dass eine erweiterte Benutzung der psychotropen Mittel mit Rücksicht auf Abschwächung der Vitalität der endogenen Depressionen, auf Verkleinerung deren Tiefe und Überführung deren typischer Phasen zu den Varietäten mit schlaffendem Verlauf die Voraussetzungen zu der Entwicklung der unausgeprägten, gegen die Psychopharmakotherapie Widerstandsfählen Depressionen der geringeren Tiefe mit sich bringt.

Eine Verkürzung der Anfallsdauer der Depression unter dem Einfluss von Antidepressanten bewirkt auch eine Abnahme der Remissionsfristen, wodurch des öfteren die Gesamtdauer des Krankhaften Zustandes bei einem periodischen Psychoseverlauf dieselbe wie auch vor der Behandlung mittels Antidepressanten unverändert bleibt. In anderen Fällen kann enn leichterer Anfallsverlauf dank der positiven Einwirkung von Antidepressinten zu$ dessen langwierigerem Vorgang führen (P.V.Birjukovich und andere "Pharmakologische Grundlagen der antidepressiven Einwirkung", 1970, Leningrad, SS. 61 bis 65; P.V. Birjukovich und andere "Zyklothyme Depression", /Zirkulyarnaya depressia"/, 1979, "Naukova Dumka", Kiev, S. 324).

Bereits ist bekannt eine Einrichtung ("Elektronenimpulsstimulator" "Stimulyator impulsny elektronny", Technische Anleitung, 1973, "Reklama", Kiev, SS. 3 bis 5), die einen Impulsgenerator und mit diesem verbundene Elektroden, Anode und Kathode, vorsieht.

Diese bekannte Einrichtung gibt jedoch keine Möglichkeit, eine getrennte Einwirkung auf die rechte und linke Gehirnshemisphäre mit verschiedenartigen Reizerregern gleichzeitig auszuüben, mit einer optpschen Bestrahlung an der Netzhaut des Menschensauges anzugreifen. Bekannt ist eine Einrichtung (Stimulator impulsny elektrodny, Technische Beschreibung, 1973, "Reklama" (Kiev), S. 3-5), die mit Elektroden mit Anode und Katode) ausgestattet ist. Bekannt ist eine Einrichtung, die einen Impulsgenerator und Elektroden vorsieht. Die bekannte Einrichtung ermöglicht es nicht, getrennt an der rechten und linken Gehirnhemisphäre mit den unterschiedlichen Reizerregern gleichzeitig einzuwirken sowie ermöglicht es nicht, die Eingriffe an der Netzhaut des Menschenauges mit einer optischen Bestrahlung auszuüben.

Bei den anderen, bisher bekannten Einrichtung (SU, A, 1303168, 1389774, 852336) werden seltkive, Subsensorelektrostimulationen an verschiedenen Gebieten des Gehirns ausgenutzt, die die Zerstörung der pathologisch beständigen Zwischenhemisphärenverhältnisse und die Formgebung einer alternativen Dominanzaktivität begünstigen, welche während langer Zeit existiert und im wesentlichen Masse den Zustand einer Person bestimmt. Im klinischen Sinne wirkt sie bei der rückwärtigen Dynamik der psychopathologischen Störungen, bei der Vergrösserung der Anzahl der dauernden Remissionen aus, es ist auch eine Veränderung der Psychosedynamik in Richtung deren günstigeren Verlaufs möglich.

Für die Korrektion des Gefühlzustandes bei den praktisch gesunden Personen, die Behandlung und Korrektion der Affektstörungen kleinerer Tiefe bei den Kranken mit den angrenzenden Psychonervenstörungen bedarf es jedoch einer anpassungsfähigen Korrektion des Gefühlzustandes, mehrmalige Anwendung der jeweiligen Einwirkung auf ein und dieselbe Person während einer dauernden Zeitperiode mit Rücksicht auf eine eventuelloft auftretende Auswechselung der Gefühlzustande und Affekte.

Darüber hinaus bestehen Kontraindikationen für die Anwendung der bekannten Einrichtungen zwecks der lateralen Physiotherapie bei den Kranken mit den chrnonischen Infektionsherden, der essentiellen Hypertonie, da sonst die Nebenerscheinungen und Komplikationen durch deren Benutzung infolge einer nicht genauen Bestimmung der Stärke, Dosis und Lateralität der auszuübenden Einwirkung sowie durch eine unfachmännische Bestimmung des psychischen Status der jeweiligen Person hervorgerufen werden.

Die erwähnten, bekannten Einrichtungen geben auch keine Möglichkeit, eine getrennte Einwirkung auf die rechte und linke Gehirnshemisphäre vermittels der verschiedenartigen Reizerreger gleichzeitig durchzuführen, an der Netzhaut der Menschensaugen mit dem Lichtstrom anzugreifen.

Bekannt ist noch eine Einrichtung zur Korrektion des Gefühlzustandes einer Person ("Handbuch für Fotografie", 1986, Technik, Kiev, A.S.Gurlev "Lichtbogen-Quecksilber-Luminiszenzlampen" - "Dugovye rtutnye ljuminestsentnye lampy", S. 72), die eine Quecksilberlampe darstellt untergebracht in eine Glasflasche, die vom Innenluminophor ummantelt ist. Diese Quecksilberlampe wird aus einer Wechselstromquelle mit einer Spannung von ll5 V, eine Stromstärke von 0,8 A gespeist. Die betreffende Quecksilberlampe strahlt einen Lichtstrom von 3400 lm.

Durch diese Einrichtung kann auch keine getrennte Einwirkung auf der rechten und linken Gehirnhemisphäre mit den verschiedenartigen Reizerregern gleichzeitig ausgeübt werden.

Offenbarung der Erfindung

Der vorliegenden Erfindung liegt die Aufgabe zugrunde, eine solche Einrichtung zur Korrektion des Gefühlzustandes einer Person zu entwickeln, die es ermöglicht auf die rechte und linke Gehirnhemisphäre gleichzeitig mit verschiedenartigen Reizerregern getrennt einzuwirken.

Dies wird dadurch erreicht, dass in der Einrichtung zur Korrektion des Gefühlzustandes einer Person, die eine Lichtquelle einschliesst, erfindungsgmmäss mindestens ein Lichtfilter mit mindestens zwei Filterhälften vorgesehen ist, die eine Grenze unter Ermöglichung deren Verlegung gleichzeitig in der ungefähr mit der Sagittalebene des Auges zusammenfallenden Ebene und in der anderen, fast der Frontalebene parallel verlaufenden Ebene trennt, wobei die erste Filterhälfte den Lichtstrom mit den aus dem Wellenlängenbereich zwischen $5,85 \cdot 10^{-7}$ m und $7,60 \cdot 10^{-7}$ m ausgewählten Wellenlängen und die andere Filterhälfte den Lichtstrom mit den aus dem Wellenlängenbereich zwischen $3,80 \cdot 10^{-7}$ m und $5,50 \cdot 10^{-7}$ m ausgewählten Wellenlängen von der Lichtquelle aus hindurchtreten lässt, die Anordnung zumindest eines Lichtfilters zwischen der Lichtquelle und dem Auge in einem an dem Auge möglichsnahen Abstand derart ermöglicht wird, dass zumindest ein Punkt an jeder Filterhälfte in einer der Frontalebene ungefähr parallelverlaufenden Ebene liegt.

Dies wird ebenfalls dadurch erreicht, dass in der Einrichtung zur Korrektion des Gefühlzustandes einer Person, die eine Lichtquelle vorsieht, erfindungsgemäss zumindest ein Lichtfilter mit zumindest zwei Filterhälften vorgesehen ist, die eine Grenze unter Ermöglichung deren Verlegung gleichzeitig in der mit der Sagittalebene des Auges zusammenfallenden Ebene und in der anderen, der Frontalebene fast parallel verlaufenden Ebene trennt, wobei die erste Filterhälfte den Lichtstrom mit den aus dem Wellenlängenbereich zwischen $5,70 \cdot 10^{-7}$ m und $6,00 \cdot 10^{-7}$ m ausgewählten Wellenlängen und die andere Filterhälfte den Lichtstrom mit den aus dem Wellenlängenbereich zwischen $5,1 \cdot 10^{-7}$ m und $5,60 \cdot 10^{-7}$ m ausgewählten Wellenlängen von der Lichtquelle aus hindurchtreten lässt, die Anordnung zumindest eines Lichtfilters zwischen der Lichtquelle und dem Auge in einem an dem Auge möglichsnahen Abstand derart ermöglicht wird, dass zumindest ein Punkt an jeder Lichtfilterhälfte in einer der Sagittalebene des Auges ungefähr senkrechten Ebene liegt.

Die wird ebenfalls dadurch gelöst, dass in der Einrichtung zur Korrektion des Gefühlzustandes einer Person, die eine Lichtquelle vorsieht, erfindungsgemäss zumindest ein Lichtfilter mit zumindest zwei Filterhälften vorgesehen ist, die eine Grenze unter Ermöglichung deren Verlegung gleichzeitig in der beinahe mit der Sagittalebene des Auges zusammenfallenden Ebene und in der anderen, der Frontalebene fast parallel verlaufenden Ebene trennt, wobei die erste Filterhälfte den Lichtstrom mit den aus dem Wellenlängenbereich zwischen $5,50 \cdot 10^{-7}$ m und $6,20 \cdot 10^{-7}$ m ausgewählten Wellenlängen und die andere Filterhälfte den Lichtstrom mit den aus dem Wellenlängenbereich zwischen $4,80 \cdot 10^{-7}$ m und $5,40 \cdot 10^{-7}$ m ausgewählten Wellenlängen von der Lichtquelle aus hindurchtreten lässt, die Anordnung zumindest eines Lichtfilters zwischen der Lichtquelle und dem Auge in einem an dem Auge möglichsnahen Abstand derart ermöglicht wird, dass mindestens ein Punkt an jeder Lichtfilterhälfte in einer der Sagittalebene beinah senkrechten Ebene liegt.

Zweckmässigerweise ist mindestens ein Lichtfilter in Form einer Haftschale auszuführen.

Möglicherweise ist die Einrichtung zur Korrektion des Gefühlzustandes einer Person mit je einem als Haftschale ausgeführten Lichtfilter für jedes Auge zu versehen, an welchen Lichtfiltern die auf der linken Seite der Filterhälftentrenngrenzen liegenden Lichtfilterhälften den Lichtstrom mit den aus einem Wellenlängenbereich ausgewählten Wellenlängen und die auf der rechten Seite der Filterhälftentrenngrenzen liegenden Lichtfilterhälften den Lichtstrom mit den aus einem anderen Wellenlängenbereich ausgewählten Wellenlängen hindurchtreten lassen.

Es ist auch die Ausstattung zumindest eines Lichtfilters mit dem Brillengestell durchaus möglich.

Sachgemäss geeignet ist die Einrichtung zur Korrektion des Gefühlzustandes einer Person mit zwei im Brillengestell erfassten Lichtfiltern, deren auf der Linksseite der Filterhälftentrenngrenzen liegende Lichtfilterhälften den Lichtstrom mit seinen aus einem Wellenlängenbereich ausgewählten Wellenlängen und die auf der rechten Seite der Filterhälftentrenngrenzen liegenden Lichtfilterhälften den Lichtstrom mit seinen aus dem anderen Wellenlängenbereich ausgewählten Wellenlängen hindurchtreten lassen.

Durch diese Ausführung der erfindungsgemässen Einrichtung zur Korrektion des Gefühlzustandes einer Person wird die Benutzung der physiologisch schwachen, adäquaten Reizerreger gesichert, eine Herabsetzung der Anzahl der Nebenerscheinungen und Komplikationen bei Anwendung der Psychopharmako- und lateralen Physiotherapie ermöglicht, eine genau Dosierung der Eingriffe bestimmt und deren Benutzung in der Psychiatrie der Grenzzustande, in der psychosomatischen Medizin, im Sport zulässig, eine Verkürzung der Behandlungsfristen der protrahierten Gefühlstörungen kleinerer Tiefe erreicht.

Die fehlenden Komplikationen und eine gute Übertragbarkeit der gleichzeitigen und abwechselnden Eingriffe der Lichtströme mit den unterschiedlichen Wellenlängen an den rechten und linken Netzhauthälften der Augen, die Entstehung einer leichten, sedativen oder euphorischen Auswirkung im Laufe des jeweiligen Eingriffs verursachen die positive, emotionalausgedrückte Reaktion des Patienten und geben die Möglichkeit, die erfindungsgemässe Einrichtung zur Korrektion des Gefühlzustandes einer Person von praktisch gesunden Leuten wie beispielsweise Operateure, Sportler, Pädagogen, die sich mit der emotionalbelasteten Tätigkeit beschäftigen, als ein Mittel des individuellen Gebrachs zur Optimisierung ihrer Stimmung und des psychischen Zustandes auszunutzen.

Kurzbeschreibung der Zeichnungen

Die erwähnten und anderen Ziele der vorliegenden Erfindung werden nachstehend an Hand der Beschreibung deren konkreter Ausführungsbeispiele und angelegten Zeichnungen näher erläutert, in denen es zeigt:

Fig. 1 erfindungsgemässe Einrichtung zur Korrektion des Gefühlzustandes einer Person in schematischer Darstellung;

Fig. 2 erfindungsgemässe Einrichtung zur Korrektion des Gefühlszustandes einer Person mit den als Haftschalen ausgeführten Lichtfiltern in schematischer Darstellung;

Fig. 3 im Brillengestell festerfasste Lichtfilter der erfindungsgemässen Einrichtung zur Korrektion des Befühlzustandes einer Person in schematischer Darstellung.

Beste Ausführungsvariante der Erfindung

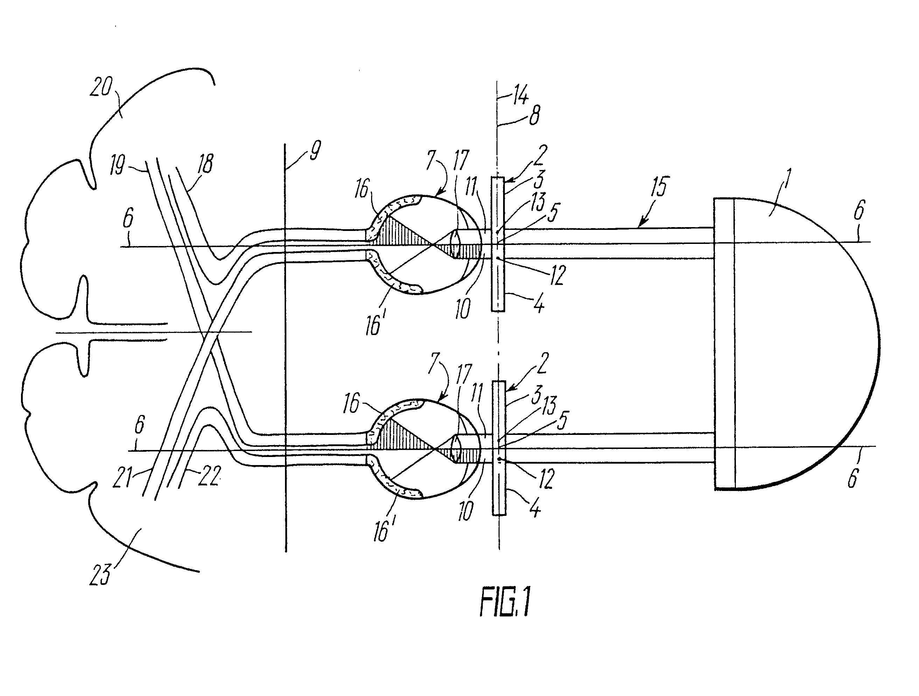

Die erfindungsgemässe Einrichtung zur Korrektion des Gefühlzustandes einer Person enthält eine Lichtquelle 1 (Fig. 1), mindestens ein Lichtfilter 2, gegebenenfalls zwei Lichtfilter 2. Jedes Lichtfilter 2 besteht mindestens aus seinen zwei Filterhälften 3, 4 die die Grenze 5 unter Ermgölichung der Verlegung gleichzeitig in einer mit der Sagittalebene 6 des Auges 7 beinahe zusammenfallenden Ebene und in der anderen, der Frontalebene 9 etwa parallel verlaufenden Ebene 8 trennt.

Durch die Filterhälfte 4 jedes Lichtfilters 2 kann ein Lichtstrom 10 von der Lichtquelle 1 aus mit den aus dem ersten Wellenlängenbereich zwischen $5{,}85 \cdot 10^{-7}$ m und $7{,}60 \cdot 10^{-7}$ m ausgewählten Wellenlängen durchlasssen, während die Filterhälfte 3 einen Lichtstrom 11 von der Lichtquelle 1 aus mit den aus einem anderen Bereich der Wellenlängen zwischen $3{,}80 \cdot 10^{-7}$ m und $5{,}50 \cdot 10^{-7}$ m ausgewählten Wellenlängen hindurchströmen lässt, wobei die Möglichkeit besteht, zumindest ein Lichtfilter 2 zwischen der Lichtquelle 1 und dem Auge 7 in einem Abstand der grösstmöglichen Annäherung an dem Auge derart anzuordnen, dass zumindest ein Punkt 12 der Filterhälfte 4 an dem Lichtfilter 2 und ein Punkt 13 oder Hälfte 3 des Lichtfilters 2 in ein und derselben der Sagittalebene 6 des Auges 7 beinahe senkrecht verlaufenden Ebene 14 liegen. In Fig. 1 fallen beide Ebenen 14 und 8 zusammen.

Die Lichtquelle 1 greift zunächst mit ihrer optischen Strahlung 15 an den linken und rechten Netzhauthälften 16 und 16' des Auges 7 mit seiner Linse 17 an.

Die linken Netzhauthälften 16 beider Augen 7 stehen über Leitungssehbahnen 18, 19 mit der linken Gehirnhemisphäre 20 in Verbindung.

Die rechten Netzhauthälften 16' beider Augen 7 werden ihrerseits durch die Leitungssehbahnen 21, 22 mit der rechten Gehirnhemisphäre 23 verbunden.

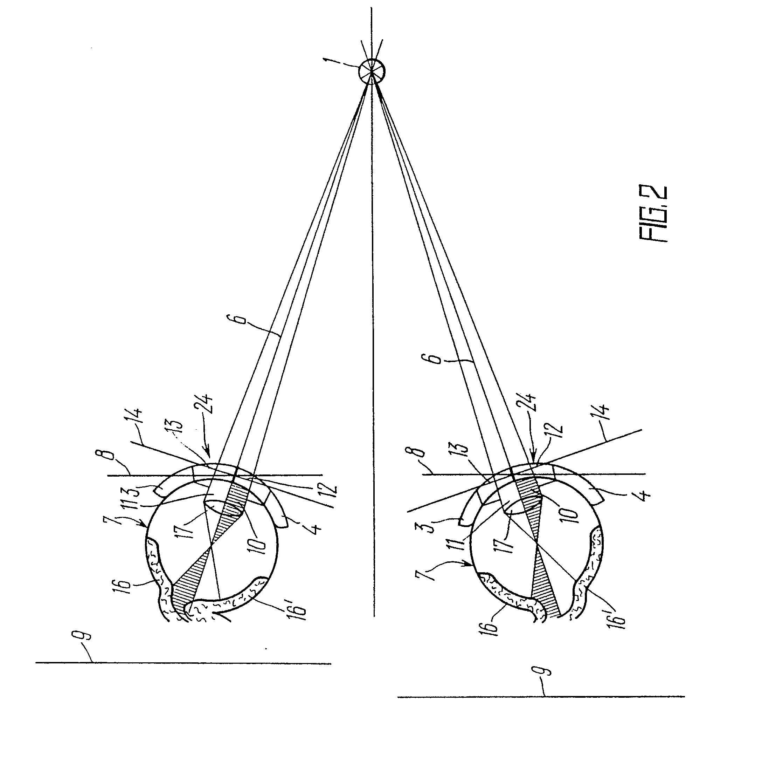

Sachgemäss möglich ist zumindest ein Lichtfilter 2 in Form einer Haftschale 24 (Fig. 2) auszuführen.

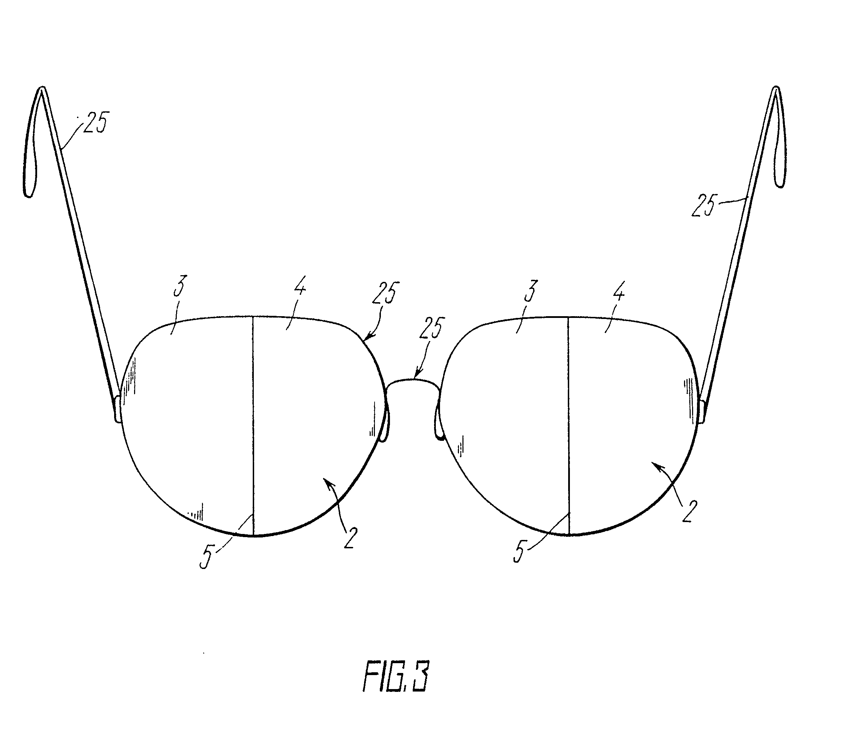

Möglicherweise ist auch zumindest ein Lichtfilter 2 mit dem Brillengestell 25 (Fig. 3) zu erfassen.

Die Wirkungsweise der beanspruchten Einrichtung zur Korrektion des Gefühlzustandes einer Person besteht erfindungsgemäss im folgenden.

Zunächst wird der Gefühlzustand einer Person mit Hilfe eines der bisher bekannten Verfahren, beispielsweise vermittels des klinischen Verfahrens, des Verfahrens der standardisierten Abschätzung der psychischen Störungen, der experimentellpsychologischen Abschätzung sowie durch Besprechung mit der praktisch gesunden Person, durch Beobachtung dessen Verhaltens, Analyse und Abschätzung der von der jeweiligen Person nach Selbstüberwachung dargelegten Angaben ermittelt.

Nach der Ermittlung des Gefühlzustandes der jeweiligen Person wird die Lichtquelle 1 (Fig. 1) mit ihrer optischen Strahlung 15 vor den Augen dieser Person gestellt. Dabei wird mindestens ein Lichtfilter 2 in dem Abstand mit einer grösstmöglichen Annäherung an dem Auge 7 zurückgehalten. Die Trenngrenze 5 der Hälften 3 und 4 des Lichtfilters 2 wird dabei in der mit der Sagittalebene 6 des jeweiligen Auges 7 beinahe zusammenfallenden Ebene und gleichzeitig in der der Frontalebene 9 etwa parallel verlaufenden Ebene 8 verlegt.

Unter diesen Umständen sind zumindest ein Punkt 12 an der Hälfte 4 des Lichtfilters 2 und zumindest ein Punkt 13 an der Hälfte 3 des Lichtfilters 2 mit einer der Sagittalebene 6 des Auges 7 beinahe senkrechten Ebene 14 vereint. Vermittels des Lichtfilters 2 wird die optischen Strahlung 15 in den Lichtstrom 10 und in den Lichtstrom 11 für jedes Auge 7 umgewandelt. Zu gleicher Zeit lässt man mit dem Lichtstrom 10 an den linken Netzhauthälften 16 jedes Auges 7 und mit dem Lichtstrom 11 auf dir rechten Netzhauthälften 16' jedes Auges 7 unter zumindest einmaliger Wiederholung der Einwirkung angreifen.

Je nach dem Gefühlzustand der Person können verschiedene Varianten der Zusammenstellung der Hälften 3, 4 des Lichtfilters 2 getroffen werden, die das Licht mit den aus den nachstehenden Bereichen ausgewählten Wellenlängen hindurchtreten lassen: $5,85 \cdot 10^{-7}$ bis $7,60 \cdot 10^{-7}$ m, $3,80 \cdot 10^{-7}$ bis $5,50 \cdot 10^{-7}$ m, $5,10 \cdot 10^{-7}$ bis $5,60 \cdot 10^{-7}$ m, $5,70 \cdot 10^{-7}$ m$^{bis}$bis $6,0 \cdot 10^{-7}$ m, $5,50 \cdot 10^{-7}$ bis $6,20 \cdot 10^{-7}$ m, $4,80 \cdot 10^{-7}$ bis $5,40 \cdot 10^{-7}$ m.

Bei den Gefühlzuständen, bei denen als Hauptzustand zumindest einer der Zustände wie Schwermut, Melancholie, Apathie, Schlaffheit, Geistesträgheit, Angst, Schreck, Ratlosigkeit, Hypochondrie, Wirklichkeitsentfremdung (erste Zustandsgruppe) vertreten wird, vor den Augen der jeweiligen Person werden die Lichtfilter 2 als erste Variante angeordnet, deren Filterhälften 4 die Lichtströme 10 mit ihren aus dem ersten Wellenlängenbereich zwischnn $5,85 \cdot 10^{-7}$ m und $7,60 \cdot 10^{-7}$ m ausgewählten Wellenlängen und die Filterhälften 3 den Lichsstrom 11 mit seinen aus einem anderen Bereich zwischen $3,80 \cdot 10^{-7}$ m und $5,50 \cdot 10^{-7}$ m ausgewählten Wellenlängen hindurchleiten. Die Einwirkung wird zumindest während, einer Zeitspanne ausgeübt, bis die persönliche, deutliche Wahrnehmung der Trenngrenzen zwischen den Lichtströmen, die Empfindung der Färbungsdifferenz verschwindet, d.h. im Durchschnitt innerhalb von 30 Sekunden bis 50 Minuten. Hiernach wird die optische Bestrahlung 15 der Lichtquelle 1 abgestellt und die Lichtquelle 1 selbst samt den Lichtfiltern 2 weggenommen.

Falls der Patient die Einwirkung der durch die Lichtfilter 2 der ersten Variante durchgelassenen Lichtströme 10, 11 als unangenehme für sich aufnimmt, werden die Lichtfilter 2 der anderen Variante vor den Augen des Patienten bei dessen Gefühlzuständen der ersten Gruppe gestellt, bei denen die Filterhälften 4 die Lichtströme 10 mit ihren aus einem Bereich zwischen $5,50 \cdot 10^{-7}$ m und $6,20 \cdot 10^{-7}$ m ausgewählten Wellenlängen und die Filterhälften 3 den Lichtstrom 11 mit seinen aus dem Bereich zwischen $4,80 \cdot 10^{-7}$ m und $5,40 \cdot 10^{-7}$ m ausgewählten Wellenlängen durchlassen. Die Einwirkung wird zumindest während der Zeitdauer ausgeübt, bis die persönliche Wahrnehmung der Farbendifferenz, verschwindet, im Durchschnitt innerhalb von 2 Minuten bis 2 Stunden, wonach die optische Bestrahlung 15 von der Lichtquelle 1 aus abgestellt und die Lichtquelle 1 selbst samt den Lichtfiltern 2 weggenommen wird.

Um das Ergebnis nach dem Eingriff der durch die Lichtfilter 2 der ersten Variante durchtretenden Lichtströme 10, 11 zu verstärken, werden vorher die Lichtfilter 2 dritter Variante vor den Augen des Patienten gestellt, bei denen die Filterhälften 4 die zusätzlichen Lichtströme 10 mit ihren aus beispielsweise drittem Bereich zwischen $5,10 \cdot 10^{-7}$ m und $5,60 \cdot 10^{-7}$ m ausgewählten Wellenlängen und die Filterhälften 3 die zusätzlichen Lichtströme 11 mit ihren aus beispielsweise viertem Bereich zwischen $5,70 \cdot 10^{-7}$ m und $6,00 \cdot 10^{-7}$ m ausgewählten Wellenlängen gleichzeitig hindurchleiten. Der Eingriff wird mindestens während der Zeitspanne ausgeübt, bis die persönliche, deutliche Wahrnehmung der Trenngrenzen zwischen den Lichtströmen (die Empfindung der Farbendifferenz) verschwindet, d.h. im Durchschnitt innerhalb von 30 Sekunden bis 15 Minuten. Danach wird die Einwirkung der durch die Lichtfilter 2 erste Variante hindurchgeleiteten Lichtströme 10, 11 ausgeübt.

Bei den Gefühlzuständen, bei denen als Hauptzustand zumindest einer der Zustände wie Unruhe, Gespannheit, Böswilligkeit, Misstrauen, Hypomanie, Zudringlichkeit, Zornigkeit (zweite Zustandsgruppe) vertreten wird, werden die Lichtfilter 2 (vierte Variante) vor den Augen des Patienten gestellt, bei denen die Filterhälften 4 die Lichtströme 10 mit den aus dem Bereich zwischen 3,80 $10^{-7}$ m und 5,50 $10^{-7}$ m ausgewählten Wellenlängen und die Filterhälften 3 die Lichtströme mit den aus dem Bereich zwischen 5,85 $10^{-7}$ m und 7,60 $10^{-7}$ m ausgewählten Wellenlängen durchlassen. Der Eingriff verläuft zumindest während einer Zeitspanne, bis die persönliche Wahrnehmung der Farbendifferenz verschwindet, d.h. im Durchschnitt innerhalb von 30 Sekunden bis 50 Minuten. Alsdann wird die optische Bestrahlung 14 von der Lichtquelle 1 aus abgeschaltet und die Lichtquelle 1 selbst samt den Lichtfiltern 2 weggenommen.

Falls der Patient den Eingriff der durch die Lichtfilter 2 vierter Variante hindurchleitenden Lichtströme 10, 11 als persönlich unangenehmer aufnimmt, werden die Lichtfilter 2 fünften Variante bei den Gefühlzuständen zweiter Gruppe vor den Augen 7 des Patienten gestellt, bei welchen Lichtfiltern 2 die Filterhälften 4 die Lichtströme 10 mit den aus dem Bereich zwischen 4,80 $10^{-7}$ m und 5,40 $10^{-7}$ m ausgewählten Wellenlängen und die Filterhälften 3 die Lichtströme 11 mit den aus dem Bereich zwischen 5,50 $10^{-7}$ und 6,20 $10^{-7}$ m ausgewählten Wellenlängen hindurchleiten.

Der Eingriff dauert mindestens während einer Zeitspanne, bis die persönliche Wahrnehmung der Farbendifferenz verschwindet, d.h. im Durchschnitt innerhalb von 2 Minuten bis 2 Stunden. Hiernach wird die optische Bestrahlung 15 von der Lichtquelle 1 aus abgestellt und die Lichtquelle 1 selbst samt den Lichtfiltern 2 weggenommen.

Um das Ergebnis des Eingriffs der durch die Lichtfilter 2 (viete Variante) hindurchleitenden Lichtströme 10, 11 zu verstärken, werden die Lichtfilter 2 (sechste Variante) vor den Augen des Patienten im voraus gestellt, deren Filterhälften 4 die zusätzlichen Lichtströme 10 mit den aus dem Bereich zwischen 5,70 $10^{-7}$ m und 6,00 $10^{-7}$ m ausgewählten Wellenlängen und die Filterhälften 3 gleichzeitig die zusätzlichen Lichtströme 11 mit den aus dem Bereich zwischen 5,10 $10^{-7}$ m und 5,60 $10^{-7}$ m ausgewählten Wellenlängen hindurchleiten.

Der Eingriff dauert dabei zumindest während einer Zeitspanne, bis die persönliche Wahrnehmung der Farbendifferenz verschwindet, d.h. im Durchschnitt von 30 Sekunden bis 15 Minuten. Alsdann wird die Einwirkung mit den durch die Lichtfilter 2 (vierte Variante) hindurchleitenden Lichtströmen 10, 11 durchgeführt.

Bei den als Haftschale 24 für jedes Auge 7 ausgeführten Lichtfiltern 2 (Fig. 2) leiten die an der linken Seiten der Trenngrenzen 5 der Filterhälften 3, 4 befindlichen Filterhälften 4 einen Lichtstrom 11 mit den aus dem ersten Bereich ausgewählten Wellenlängen und die an der rechten Seiten der Trenngrenzen 5 der Filterhälften 3, 4 befindlichen Filterhälften 3 beider Lichtfilter 2 einen Lichtstrom 10 mit den aus dem anderen Bereich ausgewählten Wellenlängen hindurch.

Falls die Einrichtung mit zwei im Brillengestell 25 (Fig. 3) erfassten Lichtfiltern 2 versehen ist, tritt der Lichtstrom 11 mit den aus dem ersten Bereich ausgewählten Wellenlängen durch die an der linken Seite der Trenngrenzen 5 der Filterhälften 3, 4 befindlichen Hälften 4 der Lichtfilter 2 (Fig. 1) und der Lichtstrom 10 mit den aus dem anderen Bereich ausgewählten Wellenlängen durch die an der rechten Seite der Trenngrenzen 5 der Filterhälften 3, 4 befindlichen Hälften der Lichtfilter 2 hindurch.

Bei den Gefühlzuständen der ersten und zweiten Gruppe werden die Lichtströme 10 in den linken Netzhauthälften 16 beider Augen 7 in die elektrischen Signale, Reizerreger umgewandelt, die in den Leitungssehbahnen 18, 19 in die linke Gehirnhemisphäre 20 geleitet werden, während zu gleicher Zeit in den rechten Netzbauthälften 16' beider Augen 7 der Lichtstrom 11 in die über die Leitungssehbahnen 21, 22 in die rechte Gehirnhemisphäre 23 zutretenden, elektrischen Signale, Reizerreger umgewandelt wird. In zumindest einer aus sechs nach der Vereinigung der Filterhälften 3, 4 bestehenden Varianten der vor den Augen 7 des Patienten angeordneten Lichtfilter 2 bewirkt der Eingriff der Lichtströme 10, 11 an den linken und rechten Netzhauthälften 16 und 16' eine Verminderung oder sogar eine Verschwindung der Gefühlzustände der ersten und zweiten Gruppe bei dem Patienten im Durchschnitt in 3 bis 12 Stunden.

Die Notwendigkeit, die wiederholten Eingriffe auszuüben, hängt mit einem konkreten Gefühlzustand der jeweiligen Person, mit deren Wunsch und Zweckmässigkeit, weitere Korrektion oderen Gefühlzustandes zu durchzuführen, zusammen.

Bei der bestehender Notwendigkeit ist der Eingriff zweckmässigerweise nicht öfter als 4mal pro Tag zu wiederholen.

Solange jeweils keine Komplikationen nach der Benutzung der erfindungsgemässen Einrichtung zur Korrektion des Gefühlzustandes bestätigt worden sind, kann diese während einer langen Zeitdauer ausgenutzt werden.

Somit hat die Entwicklung der erfindungsgemässen Einrichtung die Möglichkeit mit sich gebracht, den gleichzeitigen Eingriff mit verschiedenen Reizerregern getrennt auf der linken und rechten Gehirnhemisphäre 20 und 23 durchzuführen, wodurch die Behandlungsfristen der zyklothymischen Depression, Jahreszeitenaffektsörung herabgesetzt werden konnten, die Anzahl der Nebenerscheinungen und Komplikationen nach der Anwendung der Psychopharmakotherapie und lateralen Psychotherapie verkleinert, das Anwendungsgebiet der erfindungsgemässen Einrichtung zur Behandlung und Korrektion der Effektstörungen bei den Alkoholismus-, schizophreniekranken, bei den Kranken mit den nervenpsychischen und psychosomatischen Grenzstörungen; in der Sexopathologie; bei den praktisch gesunden Leuten wie Operateure, Sportler, Pädagogen erweitert werden konnte, die sich mit der gefühlbelasteten Tätigkeit beschäftigen.

Nachstehend werden Beispiele der klinischen Benutzung der erfindungsgemässen Einrichtung zur Korrektion des Gefühlzustandes einer Person angeführt.

Beispiel 1

Eine Kranke B, 31 Jahre alt. Nach dem Tod der Tochter ist die resktive Depression entwickelt worden. Klinisches Bild: ausgedrückter Schwermuteffekt, Hemmungsgefühl, Abschwächung der Esslust, Bewegungs- und Gedankenhemmung.

Die Kranke ist an der psychotraumatischen Situation fixiert, äussert die überwertvollen Ideen der Selgstbeschuldigung, selbstmörderischen Gedanken.

Vor der Kranken wird eine den Lichtstrom 1500 lm entwickelte Lichtquelle 1 in dem Abstand 1,3 m angeordnet. 1hr wird es vorgeschlagen, ihr Blick an einem von der Kranken weitvorn fernstehenden Punktfestzuhalten, um die Bewegungsausweite der Augen kleiner zu machen.

Hiernach werden die in einen Brillengestell 25 (Fig. 3) festerfassten Lichtfilter 2 derart angeordnet, dass dabei jede Trenngrenze 5 (Fig. 1) der Lichtströme 10, 11 für jedes Auge 7 gleichzeitig in zwei Ebenen verlegt wird, und zwar in der mit der Sagittalebene 6 des Auges 7 beinahe zusammenfallenden Ebene und in der der Frontalebene 9 etwa parallel verlaufenden Ebene 8. In diesem Zusammenhang werden die die zusätzlichen Lichtströme 10 mit den Wellenlängen zwischen $5,20 \cdot 10^{-7}$ m und $5,40 \cdot 10^{-7}$ m durchlassenden Hälften 4 der Lichtfilter 2 in Bezug auf die Kranke rechts an den Trenngrenzen 5 der Lichtströme 10, 11 und die den Lichtstrom 11 mit den Wellenlängen zwischen $5,75 \cdot 10^{-7}$ m und $5,85 \cdot 10^{-7}$ m hindurchleitenden Hälften 3 derselben Lichtfilter 2 in Bezug auf die Kranke links an den Trenngrenzen 5 der Lichströme 10, 11 angeordnet. Man schaltet die Lichtquelle 1 ein.

Eine gleichzeitige Einwirkung der zusätzlichen Lichtströme 10 an den linken Netzhauthälften 16 der Augen 7 und der Lichtströme 11 an den rechten Netzhauthälften 16' beider Augen 7 verläuft innerhalb 3 Minuten. In 2 Minuten nach Beginn der Einwirkung teilte die Kranke mit, dass die Grenze zwischen der 2"grünen" und "gelben" Sehfeldhälften verschwommen und in dem Sehfeld nur eine "gelblichgrüne" Farbe überlegen ist.

Nach der Beendigung der Einwirkung werden die Lichtfilter 2 mit ihren Hälften 3, 4 gewählt, durch welche ein Lichtstrom 10 mit den nach Worten der Kranken grösstangenehmen Wellenlängen zwischen $6,70 \cdot 10^{-7}$ m und $7,20 \cdot 10^{-7}$ m und ein Lichtstrom 11 mit den nach Worten der Kranken grösstangenehmen Wellenlängen zwischen $4,40 \cdot 10^{-7}$ m und $4,70 \cdot 10^{-7}$ m tritt.

Dadurch wurden die genannten Lichtfilter 2 durch die Lichtfilter 2 mit ihren Hälften 4 und 3 ersetzt, welche die Lichtströme 10, 11 mit den aus den Bereichen ziischen $5,85 \cdot 10^{-7}$ m und $7,60 \cdot 10^{-7}$ m sowie zwischen $3,80 \cdot 10^{-7}$ m und $5,50 \cdot 10^{-7}$ m hindurchleiten. Diese in dem Brillengestell 25 (Fig. 3) festumfassten Lichtfilter 2 wurden wie oben beschrieben ähnlich angeordnet.

Nach dem Einschalten der Lichtquelle 1 (Fig. 1) greifen die Lichtströme 10 an den linken und die Lichtströme 11 an den rechten Netzhauthälften 16 und 16' beider Augen gleichzeitig an. In 1 Minute und 20 Sekunden teilte die Kranke mit, dass die Trenngrenze zwischen der "roten" und "blauen" Sehfeldhälften eine "Himbeer"-Schattierung erhält, an ihrer Schärfe verliert und sich unter Ausfüllen des Mittelabschnitts des Sehfeldes zu rechts und links allmählich ausdehnt. Diese Einwirkung verläuft 12 Minuten, wonach die Lichtquelle 1 abgeschaltet, das Brillengestell 25 (Fig. 3) mit den Lichtfiltern 2 (Fig. 1) von der Kranken weggenommen wird. Im Laufe der Einwirkung hat die Kranke eine auftretende leichte Erschlaffung, eine Verkleinerung der Gedanken- und Bewegungshemmung bestätigt. In 30. bis 40. Minute nach der Beendigung der Einwirkung haben sich alle diesen Veränderungen des Zustandes der Kranken allmählich erlöscht und im klinischen Sinne kehrte der Gefühlzustand zu diesem vor der Einwirkung nah zurück. In 7 Stunden nach der Beendigung der Einwirkung hat die Kranke aus Eigenwillen dem medizinischen Personal bei dem Raumsaubermachen, der Krankenernährung zu helfen begonnen. Die Gedanken- und Bewegungshemmung ist verschwunden, die Schwermut und der Hemmungszustand nahmen im wesentlichen ab, die Kranke hat an den Besprechungen anderer Kranken teilgenommen, auf die Scherzen geantwortet.

Am nächsten Tag sind die erwähnten Veränderungen des Zustandes der Kranken verschwunden. Klinisches Bild war nach wie vor: Schwermutaffekt, Hemmungsempfindung, Bewegungshemmung, Appetit ist vergangen. Die Kranke ist an der psychotraumatischen Situation fixiert, trotzdem sind die selbstmörderischen Gedanken und die überwertvollen Ideen der Selgstbeschuldigung ein wegig verschwunden. Nach dem Wunsch der Kranken sind 14 wiederholte Einwirkung der Lichtströme 10, 11 auf die Netzhaut beider Augen 7 durchgeführt. Die genannten Einwirkungen wurden 14mal vorgenommen. Die Kennwerte und der Vorgangsverlauf werden in die Tabelle 1 zusammengeführt.

Nach dem 12. Eingriff ist die psychopathologische Symptomatik wie Schwermut, Hemmungsempfinden verschwunden, nun kommt die Kritik eigener, früher geäusserter, pathologischer Ideen. Es treten normaler Schlaf und Appetit auf.

Bei der betreffenden Heilungskorrektion des Gefühlzustandes vermittels der erfindungsgemässen Einrichtung werden keine dabei entstandenen Nebenerscheinungen und Komplikationen bei dieser Kranken durch die Benutzung der Psychopharmakotherapie vermerkt, wie sie in den bekannten Veröffentlichungen beschrieben werden.

Diese Kranke ist bei einem befriedigenden Zustand gesundgeschrieben und setzte ihre Tätigkeit an dem vorherigen Arbeitsplatz. Im Laufe der Dispensairverlaufsbeobachtung ist sie während über zwei Jahre zu dem Psychiater nicht zugegangen, führte keine Beschwerden, es wurde keine psychopathologische Simptomatik nachgewiesen, sie ist aus der Kontrolle des Psychiaters ausgeschlossen.

Tabelle I

| Lfd. Nr. der Einwirkung | Lfd. Nr. des Tages | Lichtstrom der Lichtquelle I in [lm] | Abstand der Lichtquelle I von den Augen 7 in [m] | Dauer der Einwirkung in [min] | Grenzen der gewählten Wellenlängen der Ströme 10,11, die angreifen an: | |
|---|---|---|---|---|---|---|
| | | | | | linken Netzhauthälften 16 beider Augen 7 in $[10^{-7} m]$ | rechten Netzhauthälften beider Augen 7 in $[10^{-7} m]$ |
| 1 | 1 | 1500 | 1,3 | 3,0 | 5,20-5,40 | 5,75-5,85 |
| 2 | 1 | 1800 | 1,0 | 12,0 | 6,70-7,20 | 4,40-4,70 |
| 3 | 2 | 1500 | 1,2 | 4,0 | 5,20-5,50 | 5,75-5,85 |
| 4 | 2 | 1800 | 0,95 | 15,0 | 6,0-7,40 | 4,00-4,50 |
| 5 | 3 | 1700 | 1,1 | 5,0 | 5,10-5,40 | 5,75-5,85 |
| 6 | 3 | 2000 | 1,0 | 20,0 | 5,90-6,40 | 4,60-4,80 |
| 7 | 5 | 1600 | 1,3 | 5,0 | 5,10-5,40 | 5,75-5,85 |
| 8 | 5 | 1800 | 1,2 | 20,0 | 6,00-6,60 | 4,50-4,80 |
| 9 | 6 | 1800 | 1,0 | 3,0 | 5,20-5,50 | 5,80-5,90 |
| 10 | 6 | 2100 | 0,9 | 12,0 | 6,60-7,40 | 4,80-5,00 |
| 11 | 8 | 1600 | 1,2 | 2,0 | 5,20-5,40 | 5,80-5,90 |
| 12 | 8 | 1900 | 1,0 | 8,0 | 6,20-7,00 | 5,00-5,30 |
| 13 | 11 | 1400 | 1,0 | 1,5 | 5,10-5,40 | 5,80-5,90 |
| 14 | 11 | 1000 | 1,0 | 6,0 | 5,80-6,10 | 4,90-5,10 |

Beispiel 2

Der Kranke B, 32 Jahre alt, leidet unter der Zyklothymie. Beschwerden über Schwermut, Gleichgültigkeit gegen die Arbeit, Vergnügungen, über die erhöhte Ermüdbarkeit, Zunahme der Körpersmasse. Im klinischen Sinne sind die Energielosigkeit , Abschwächung des Gefühlkontaktes. Neigung zu der pessimistischen Auslegung der Ereignisse, Abnahme der Schnelligkeit bei den Gedankenvorgängen, die Sprach- und Bewegungshemmung zu vermerken. Der Erkrankungslauf zeichnet sich durch zyklische Auswechselungen der Herbst-Winterdepressionen gegen die Frühlings-Sommer-Hypomanien oder -euthymien.

Im betreffenden Beispiel werden alle Handlungen diesen im Beispiel 1 des Gebrachts der erfindungsgemässen Einrichtung vorgeführten gemäss durchgeführt. Insgesamt sind 8 Eingriffe wie in Beispiel 1 ähnlich vorgenommen, deren Zustandskennwerte in der Tabelle 2 zusammengestellt sind.

Nach 8. Heileingriff ist die Behandlung in Anbetracht der vollkommenen Reduktion der Affektstörungen eingestellt. Die nächste Verschlechterung des Gefühlzustandes in 40 Tage nach der durchgeführten Behandlung wurde auf die Bitte des Kranken schon in 4 Tagen etwa in derselben Verfahrensfolge der Heilungseingriffe korrigiert.

Der Kranke ist in einem befriedigenden Zustand gesundgeschrieben.

Bei der Heilungskorrektion des Gefühlzustandes vermittels der erfindungsgemässen Einrichtung sind bei dem betreffenden Kranken keine Nebenerscheinungen und Komplikationen bestätigt worden, wie sie in den bekannten Veröffentlichungen bei der Anwendung der Psychopharmakotherapie beschrieben werden.

Tabelle 2

| Lfd. Nr. des Eingriffs | Lfd. Nr. des Tages | Lichtstrom der Lichtquelle in [m] | Abstand der Lichtquelle von den Augen 7 in [m] | Dauer des Eingriffs [min] | Grenzen der gewählten Wellenlängen der Lichtströme IO, II, die angreifen an: | |
|---|---|---|---|---|---|---|
| | | | | | der linken Netzhauthälften bei der Augen 7 in $[10^{-7}\,m]$ | rechten Netzhauthälften beider Augen 7 in $[10^{-7}\,m]$ |
| I | I | I400 | I,2 | 5,0 | 5,00–5,30 | 5,75–5,85 |
| 2 | I | 2000 | I,0 | 20,0 | 5,90–6,40 | 4,70–5,00 |
| 3 | 2 | I600 | I,2 | 7,0 | 5,I0–5,40 | 5,40–5,60 |
| 4 | 2 | 2I00 | I,0 | 30,0 | 6,60–7,40 | 4,30–4,70 |
| 5 | 3 | I700 | I,2 | 6,0 | 5,00–5,30 | 5,75–5,I5 |
| 6 | 3 | 2200 | I,0 | 25,0 | 6,00–6,70 | 4,70–4,90 |
| 7 | 5 | I600 | I,2 | 4,0 | 5,00–5,20 | 5,80–5,90 |
| 8 | 5 | 2I00 | I,0 | I7,0 | 5,90–6,I0 | 4,90–5,I0 |

Beispiel 3

Der Kranke G, 40 Jahre alt, leitet unter der langsam progredienten Schizophrenie mit überlegenden Affektstörungen.

Klinisches Bild: die Affektstörungen sind in Form von gehemmten Depressionen, überwiegend den obsessiven und phobischen transitorischen Störungen zum Ausdruck gekommen. Beim Beschau ist der Kranke unruhig, gespannt, mit dem Misstrauen erfasst, kann seinen Blick an einem Ort selbst für kurze Zeit nicht zurückhalten, seine Augen schimmern, "laufen von Seite zuf Seite". Nach der Zustimmung des Kranken sind an dessen Augen 7 die in Form von Haftschalen 23 (Fig. 2) ausgeführten Lichtfilter 2 (Fig. 1) mit deren die Lichtströme 10 mit den Wellenlängen zwischen 6,70 $10^{-7}$ m und 7,60 $10^{-7}$ m hindurchleitenden Hälften 4, welche Wellenlängen aus ihrem Bereich zwischen 5,85 $10^{-7}$ m und 7,60 $10^{-7}$ m ausgewählt werden, und deren die Lichtströme 11 mit den aus dem Wellenlängenbereich zwischen 3,80 $10^{-7}$ m und

4,30 $10^{-7}$ m ausgewählten Wellenlängen zwischen 3,80 $10^{-7}$ m und 5,50 $10^{-7}$ durchlassenden Hälften 3 angelegt.

Hierbei wird je eine Trenngrenze 5 der Filterhälften 3, 4 an jedem Lichtfilter 2 jeweils in der Sagittalebene 6 beider Augen 7 verlegt. Die Filterhälften 4 sollen dabei in Bezug auf den Kranken rechts an den Trenngrenzen 5 der Filterhälften 3, 4 und die Filterhälften 3 in Bezug auf den Kranken links an den Trenngrenzen 5 der Filterhälften 3, 4 liegen.

Bei ihrem Einschalten ist die die optische Strahlung 15 mit dem Lichtstrom 800 Im herausströmende Lichtquelle 1 vor den Augen 7 des Patienten mit einem Abstand zwischen 1,0 m und 1,5 m derart angeordnet, dass diese optische Strahlung 15 keine unangenehmen Empfindungen bei dem Kranken hervorruft. Der Kranke sollte nach der Empfehlung gegen die Lichtquelle 1 sehen. Nun sind jeweilige Einwirkungen mit dem Lichtstrom 10 an den linken Netzhauthälften 16 beider Augen 7 und mit dem Lichtstrom 11 an den rechten Netzhauthälften 16' beider Augen 7 während 30 Sekunden ausgeübt. In 30 Sekunden nach Beginn der betreffenden Einwirkung teilte der Kranke mit, dass die Trenngrenze ihre Deutlichkeit verloren hat, zerfliessen ist und im Sehfeld die Himbeer- und Rosafarben herrschen. Nach der Beendigung des Eingriffs sind die Lichtquelle 1 und die in Form von Haftschalen 24 (Fig. 2) ausgeführten Lichtfilter 2 (Fig. 1) von den Augen des Kranken weggenommen.

Nach dem durchgeführten Lichteingriff bestätigte der Kranke seine "Gesundheitsverbesserung", Abnahme der Aufregungsstärke, der inneren Gespanntheit. Das Misstrauen ist zurückgetreten, der Kranke benimmt sich ruhig, gesellig. Dieser Gefühlzustand bleibt während etwa einer Stunde unverändert, wonach bei dem Zustand des Kranken die depressivgefärbte Unruhe, Gespanntheit zu überlegen beginnen. Trotzdem nahmen die Unruhe und Gespanntheit in 3 bis 4 Stunden nachher wiederum allmählich bis zum ganzen Verschwinden ab, der Kranke hat sich beruhigt, nach seinen Worten "innerlich entspannt". Am nächsten Tag teilte der Kranke über die Wiederherstellung des Unruhe- und Gespanntheitszustandes bei ihm von sich selbst mit und äusserte seinen Wunsch, den Lichteingriff zu wiederholen. Es sind jetzt 12 Lichteingriffe ausgeübt, deren Verfahrenskennwerte in der Tabelle 3 zusammengestellt sind. Jeder Eingriff dauerte nicht minder als die Zeitspanne, in deren Laufe die subjektive Differenz zwischen den Farben verschwindet.

Nach 5. Eingriff wurden die unruhigdepressiven Störungen schwächer. Nach 12. Lichteingriff ist der Kranke infolge der Beseitigung der obenerwähnten Simptomatik gesundgeschrieben.

Bei der Korrektion des Gefühlzustandes bei dem betreffenden Kranken vermittels der erfindungsgemässen Einrichtung sind keine Nebenerscheinungen und begleitende Komplikationen nachgewiesen, wie sie in den bekannten Veröffentlichungen bei Anwendung der Psychlpharmakotherapie und lateralen Physiotherapie beschrieben werden.

Tabelle 3

| Lfd. Nr. des Ein- griffs | Lfd. Nr. des Ta- ges | Licht- strom der Licht- quelle I in [lm] | Abstand der Licht- quelle von den Augen 7 in [m] | Dauer des Ein- griffs in min | Grenzen der gewählten Wellenlängen der Lichtströme IO, II, die angreifen an: | |
|---|---|---|---|---|---|---|
| | | | | | linken Netz- hauthälften I6 beider Augen 7 in $[\mathrm{IO}^{-7}\,m]$ | rechten Netz- hauthälften I6' beider Augen 7 in $[\mathrm{IO}^{-7}\,m]$ |
| I | I | 800 | I,5 | 0,5 | 4,50–4,80 | 6,30–6,70 |
| 2 | 2 | 900 | I,3 | I,0 | 4,30–4,60 | 6,50–7,20 |
| 3 | 2 | 700 | I,5 | 2,0 | 3,80–4,30 | 6,70–7,60 |
| 4 | 3 | IOOO | I,3 | 4,0 | 4,50–4,80 | 6,20–6,60 |
| 5 | 3 | 700 | I,5 | 6,0 | 4,30–4,60 | 6,50–7,20 |
| 6 | 5 | 800 | I,3 | 0,5 | 5,50–4,80 | 5,90–6,20 |
| 6 | 6 | I300 | I,0 | I,0 | 4,30–4,60 | 6,30–6,70 |
| 8 | 6 | I300 | I,0 | 2,0 | 4,30–4,60 | 6,50–7,20 |
| 9 | 6 | 700 | I,3 | 4,0 | 3,80–4,30 | 6,70–7,60 |
| IO | 8 | 800 | I,3 | 6,0 | 4,30–4,60 | 6,50–7,20 |
| II | IO | IOOO | I,0 | 4,0 | 5,00–5,30 | 5,75–5,85 |
| I2 | I2 | I500 | 0,95 | 4,0 | 4,50–4,80 | 5,90–7,20 |

Beispiel 4

Der Kranke D, 40 Jahre alt. Diagnose: chronischer Alkoholismus im 2. Staiium, eine pseudoperiodische Betrunktheitsform der Trunksucht, zweite Phase des alkoholischen Abstinenzsyndroms. Zum Beschauaugenblick führte der Kranke seine Beschwerden über eine starke Neigung zu den Spirituosen, über die Kopfschmerzen und Schmerzen im Herzgebiet, über Morgenserbrechen, Schlaflosigkeit. Klinisches Bild: mit Reiz, Gespanntheit und Unruhe behaftet.

Neben der Dosintoxikationstherapie beginnt die Behandlung mit Hilfe der erfindungsgemässen Einrichtung. In diesem Zusammenhang werden alle in dem betreffenden Beispiel vermittels der erfindungsgemässen Einrichtung zustandegekommenen Handlungen wie im obenbeschriebenen Beispiel 1 ähnlich durchgeführt. Insgesamt sind 9 Eingriffe ausgeübt, deren Verfahrenskennwerte in der Tabelle 4 zusammengeführt sind.

In Hinsicht auf die ausgedrückte Labilität des Gefühlzustandes des betreffenden Alkoholismuskranken, insbesondere in ersten Tagen der Postabstinenzperiode sind die Eingriffe unter Berücksichtigung des Hauptgefühlzustandes ausgeübt. Nach 6. Heileingriff sind die Unruhe und Gespanntheit aus dem Gefühlzustnd des Kranken verschwunden. Die Heilkorrektion der Schwermut-Depressionsstörungen in dem Gefühlzustand des Alkoholismuskranken ist schon nach 9. Eingriff erfolgreich beendet.

Solange ausser der Dosintoxikationstherapie und der Korrektion des Gefühlzustandes mit Hilfe der erfindungsgemässen Einrichtung keine Psychopharmakotherapie und keine laterale Physiotherapie des betreffenden Kranken durchgeführt wurde, sind auch in diesem Beispiel keine Nebenerscheinungen und begleitenden Komplikationen nachgewiesen, wie sie bei der Anwendung der Psychopharmakotherapie und der lateralen Physiotherapie getroffen werden und in den bekannten Veröffentlichungen beschrieben sind.

Tabelle 4

| Lfd. Nr. des Eingriffs | Lfd. Nr. des Tages | Lichtstrom der Lichtquelle in $[lm]$ | Abstand der Lichtquelle von den Augen 7 in $[m]$ | Dauer des Eingriffs in $[min]$ | Grenzen der gewählten Wellenlängen der Lichtströme IO, II, die angreifen an: | |
|---|---|---|---|---|---|---|
| | | | | | linken Netzhauthälften I6 beider Augen in $[10^{-7}\,m]$ | rechten Netzhauthälften I6' beider Augen in $[10^{-7}\,m]$ |
| I | I | I800 | I,5 | 20,0 | 4,90–5,I0 | 6,80–7,40 |
| 2 | 2 | I000 | 0,9 | 35,0 | 4,80–5,00 | 6,00–6,30 |
| 3 | 2 | I400 | I,2 | 50,0 | 4,00–4,60 | 6,90–7,60 |
| 4 | 3 | I800 | 0,9 | 35,0 | 6,20–6,60 | 5,I0–5,30 |
| 5 | 3 | I600 | I,I | 50,0 | 4,60–4,90 | 6,20–6,80 |
| 6 | 5 | 2000 | 0,9 | 35,0 | 5,80–6,20 | 5,20–5,50 |
| 7 | 4 | I400 | I,2 | 20,0 | 4,80–5,00 | 6,00–6,30 |
| 8 | 5 | 2000 | 0,9 | 20,0 | 5,90–6,20 | 5,I0–5,30 |
| 9 | 7 | 2000 | 0,9 | 20,0 | 6,00–6,30 | 5,20–5,50 |

Beispiel 5

Der Kranke A., 48 Jahre alt. Während 10 Jahre leidet unter hypertonischer Krankheit in 2. Stufe. Klinisches Bild: auf dem Hintergrund der ausgedrückten Gefühllabilität sind Unruhe-Depressions- und asthenohypochondrische Störungen nachgewiesen. Während Beschau führte Beschwerden über seine erhöhte Unruhe, Reizerregtheit, vorzeitigen Samenerguss in der Friktionsperiode nach 5 bis 10 Fraktionen. Die Errektion aber bleibt erhalten. Urologisch gesund. Aus den vorgeschlagenen Lichtfiltern hat er sofort das Lichtfilter mit seiner die Lithströme der blaue und roten Farbe durchlässenden Hälften abgelehnt, indem er gesagt hat: "Rote Farbe erregt bei mir Reiz".

Auch in diesem Beispiel sind alle Handlungen solchen ähnlich ausgeübt, wie sie in dem Beispiel 1 der Benutzung der erfindungsgemässen Einrichtung beschrieben sind. Insgesamt sind 7 Eingriffe ausgeübt, deren Verfahrenskennwerte in der Tabelle 5 zusammengeführt sind.

13

Tabelle 5

| Lfd. Nr. des Eingriffs | Lfd. Nr. des Tages | Lichtstrom der Lichtquelle in [lm] | Abstand der Lichtquelle von den Augen 7 in [m] | Dauer des Eingriffs in [min] | Grenzen der gewählten Wellenlängen der Lichtströme IO, II, die angreifen an: | |
|---|---|---|---|---|---|---|
| | | | | | linken Netzhauthälften I6 beider Augen 7 in $[10^{-7}\,m]$ | rechten Netzhauthälften I6' beider Augen 7 in $[10^{-7}\,m]$ |
| 1 | 1 | 1000 | 1,0 | 4,0 | 5,10-5,70 | 5,70-5,80 |
| 2 | 2 | 1000 | 1,0 | 6,0 | 5,20-5,40 | 5,75-5,85 |
| 3 | 4 | 1500 | 1,0 | 1,0 | 5,75-5,85 | 5,0-5,30 |
| 4 | 4 | 1200 | 1,0 | 4,0 | 4,90-5,20 | 5,80-6,0 |
| 5 | 5 | 1700 | 1,0 | 1,5 | 5,85-6,20 | 5,00-5,20 |
| 6 | 5 | 1400 | 1,0 | 6,0 | 5,10-5,30 | 6,20-6,90 |
| 7 | 7 | 1800 | 1,0 | 6,0 | 5,20-5,40 | 6,40-7,00 |

Nach den durchgeführten Eingriffe ist der Gefühlzustand des Patienten zu seiner Normalität zurückgekehrt, es sind die Unruhe, Reizerregtheit sowie die astenohypochondrischen Störungen verschwunden. Nach 4. Eingriff ist die Friktionsperiode bis auf 60, nach 7. Eingriff bis auf 75-90 Friktionen verlängert, was den Gatten zugute gekommen ist und die innerliche Familienverhältnisse geregelt hat. Im weiteren wurden die kurzzeitigen Eingriffskuren nach Wunsch des Patienten sowie bei Notwendigkeit mit Hilfe der erfindungsgemässen Einrichtung durchschnittliche je 1. Kur in 1,5 bis 4 Monaten durchgeführt. Alle Eingriffe sind mit Rücksicht auf den Hauptzustand in der gesamten Struktur der Gefühlzustände des Patienten ausgeübt.

Beispiel 6

Eine Person A, 30 Jahre alt, ist als Lehrkraft auf dem Gebiet Informations- und Rechentechnik tätig, verheiratet, hat 2 Kinder, treibt Sport, insbesondere Schachspiel und Tennis aktiv. Er vermerkt die Empfindung einer Unsicherheit, manchmal auch Angst. Nach der klinischen Untersuchung ist praktisch gesund geschrieben.

In den letzten 3 Jahren ist praktisch ununterbrochen in dem Gefühlsinne stark belastet, was auf die Arbeit als Operateur im Rechenzentrum, als Lehrkraft, Sportler, als aktiver gesellschaftspolitischer Funktionär zurückzuführen ist.

Mehrmals vor Beginn der Wettkämfe trat bei ihm die Empfindung von Angst, Bewegungsgehemmtheit vor dem bevorstehenden Tennisspiel auf, die immerhin eine Niederlage bewirken.

In manchen Fällen entstehen auch Ermüdung, Reizbarkeit nach einer längeren Arbeit an der Rechenmaschine mit dem grossen Informationsmufang und demzufolge auch Fehler bei der Zusammenstellung eines Programms.

Als Lehrkraft vermerkt er vor seiner beginnenden Tätigkeit mit der neuen Schulgruppe des öfteren die Empfindung der Unsicherheit und einigermassen die Fassungslosigkeit.

Nach der vorläufigen Bekanntmachung der Person mit der erfindungsgemässen Einrichtung sind alle Eingriff auch in dem betreffenden Beispiel im Einklang mit diesen ausgeübt, wie sie in dem Beispiel 1 des Gebrauchs der erfindungsgemässen Einrichtung beschrieben sind. Die Verfahrenskennwerte der erwähnten Eingriffe sind in der Tabelle 6 zusammengestellt.

Tabelle 6

| Lfd. Nr. des Eingriffs | Lfd. Nr. des Tages | Lichtstrom aus der Lichtquelle in [lm] | Abstand der Lichtquelle von den Augen 7 in [m] | Dauer des Eingriffs 7 [min] | Grenzen der gewählten Wellenlängen der Lichtströme IO, II, die angreifen an: | |
|---|---|---|---|---|---|---|
| | | | | | linken Netzhauthälften I6 beider Augen 7 in [$10^{-7}$ m] | rechten Netzhauthälften I6' beider Augen 7 in [$10^{-7}$ m] |
| 1 | 1 | 1800 | 1,0 | 3,0 | 6,70-7,20 | 4,40-4,60 |
| 2 | 4 | 2100 | 1,0 | 2,5 | 6,90-7,60 | 3,80-4,20 |

Bei dem Patienten ist sein normaler Gefühlzustand zurückgekehrt, sind die Unsicherheit und Angst verschwunden.

Im weiteren wurden die Eingriffe nach Wunsch des Patienten bei Notwendigkeit mit Hilfe der erfindungsgemässen Einrichtung wiederholt ausgeübt.

Dadurch ermöglicht die erfindungsgemässe Einrichtung zur Korrektion des Gefühlzustandes einer Person die Eingriffe getrennt an der rechten und linken Gehirnhemisphäre mit den unterschiedlichen Reizerregern gleichzeitig, und zwar mit den Lichtströmen mit ihren aus den verschiedenen Wellenlängenbereichen ausgewählten Wellenlängen gleichzeitig getrennt an den rechten und linken Netzhauthälften der Augen durchzuführen.

Industrielle Verwertbarkeit

Die vorliegende Erfindung kann in der Psychiatrie und Narkologie zur Behandlung der Zyklothymie-, Schizophreni-, Neurosen-, Alkoholismuskranken und Kranken mit den Jahreszeiten affektstörungen; in der Sexopathologie; zur Heilkorrektion der Gefühlsstörungen bei den Kranken mit den angrenzenden Psychonervenstörungen sowie im Gebrauch von den praktisch gesunden Leuten wie Operateure, Sportler, Pädagogen, die sich mit einer gefühlsbelasteten Tätigkeit beschäftigen; bei Behandlung von Personen mit den psychosomatischen Störungen ihre Anwendung finden.

**Patentansprüche**

1. Einrichtung zur Korrektion das Gefühlzustandes einer Person, die eine Lichtquelle (1) vorsieht, dadurch **gekennzeichnet,** dass sie mindestens ein Lichtfilter (2) mit mindestens zwei Hälften (3, 4) aufweist, die eine unter Ermöglichung deren Verlegung gleichzeitig in einer fast mit der Sagittalebene (6) des Auges (7) zusammenfallenden Ebene und in der der Frontalebene (9) beinahe parallel verlaufenden Ebene (8) vorgesehene Trenngrenze (5) trennt, wobei die Filterhälfte (4) den Lichtstrom (10) aus der Lichtquelle (1) mit den aus dem ersten Wellenlängenbereich zwischen 5,85 $10^{-7}$ m und 7,60 $10^{-7}$ m ausgewählten Wellenlängen und die Filterhälfte (3) den Lichtstrom (11) aus der Lichtquelle (1) mit den aus dem zweiten Wellenlängenbereich zwischen 3,80 $10^{-7}$ m und 5,50 $10^{-7}$ m ausgewählten Wellenlängen unter Ermöglichung der Anordnung zumindest eines Lichtfilters (2) zwischen der Lichtquelle (1) und dem Auge (7) in einem Abstand der grösstmöglichen Annäherung zu dem Auge (7) derart durchlässt, dass mindestens ein Punkt (12 oder 13) jeder Hälfte (4 oder 3) des Lichtfilters (2) in einer der Sagittalebene (6) fast senkrechten Ebene (14) liegt.

2. Einrichtung zur Korrektion des Gefühlzustandes einer Person, die eine Lichtquelle (1) vorsieht, dadurch **gekennzeichnet,** dass sie mindestens ein Lichtfilter (2) mit mindestens zwei Hälften (3, 4) aufweist, die eine unter Ermöglichung deren Verlegung gleichzeitig in einer fast mit der Sagittalebene (6) des Auges (7) zusammenfallenden Ebene und in der der Frontalebene (9) beinahe parallel verlaufenden Ebene (8) vorgesehene Trenngrenze (5) trennt, wobei die Filterhälfte (4) den Lichtstrom (10) aus der Lichtquelle (1) mit den aus dem vierten Wellenlängenbereich zwischen 5,7 $10^{-7}$ m und 6,00 $10^{-7}$ m

ausgewählten Wellenlängen und die Filterhälfte (3) den Lichtstrom (11) aus der Lichtquelle (1) mit den aus dem dritten Wellenlängenbereich zwischen 5,10 $10^{-7}$ m und 5,60 $10^{-7}$ m ausgewählten Wellenlängen unter Ermöglichung derartiger Anordnung zumindest eines Lichtfilters (2) zwischen der Lichtquelle (1) und dem Auge (7) in einem Abstand der grösstmöglichen Annäherung zu den Augen (7) durchlässt, dass zumindest ein Punkt (12 oder 13) an jeder Hälfte (4 oder 3) des Lichtfilters (2) in einer fast der Sagittalebene (6) des Auges (7) senkrechten Ebene (14) liegt.

3. Einrichtung zur Korrektion des Gefühlszustandes einer Person, die eine Lichtquelle (1) vorsieht, dadurch **gekennzeichnet,** dass sie mindestens ein Lichtfilter (2) mit mindestens zwei Hälften (3, 4) aufweist, die eine unter Ermöglichung deren Verlegung gleichzeitig in der mit der Sagittalebene (6) des Auges (7) fast zusammenfallenden Ebene und in der der Frontalebene (9) beinahe parallel verlaufenden Ebene (8) vorgesehene Trenngrenze (5) trennt, wobei die Filterhälfte (4) aus der Lichtquelle (1) den Lichtstrom (10) mit seinen aus dem fünften Wellenlängenbereich, zwischen 5,50 $10^{-7}$ m und 6,20 $10^{-7}$ m ausgewählten Wellenlängen und die Filterhälfte (3) aus der Lichtquelle (1) den Lichtstrom mit seinen aus dem sechsten Wellenlängenbereich zwischen 4,80 $10^{-7}$ m und 5,40 $10^{-7}$ m ausgewählten Wellenlängen unter Ermöglichung derartiger Anordnung zumindest eines Lichfilters (2) zwischen der Lichtquelle (1) und dem Auge (7) in einem Abstand der grösstmöglichen Annäherung zu dem Auge (7) durchlässt, dass zumindest ein Punkt (12 oder 13) auf jeder Filterhälfte (4 oder 3) des Lichtfilters (2) in einer der Sagittalebene (6) des Auges (7) fast senkrechten Ebene (14) liegt.

4. Einrichtung zur Korrektion des Gefühlszustandes einer Person nach Anspruch 1 oder 2 oder 3, dadurch **gekennzeichnet,** dass mindestens ein Lichtfilter (2) als Halftschalte (24) ausgeführt ist.

5. Einrichtung zur Korrektion des Gefühlszustandes einer Person nach Anspruch 1 oder 2 oder 3, dadurch **gekennzeichnet,** dass sie für jedes Auge (7) je ein als Haftschale (24) ausgeführtes Lichtfilters (2) aufweist, wobei die an der linken Seite der Trenngrenzen (5) der Filterhälften (3, 4) liegenden Hälften (3) der Lichtfilter (2) den Lichtstrom (11) mit den aus einem Wellenlängenbereich ausgewählten Wellenlängen und die an der rechten Seite der Trenngrenzen (5) der Filterhälften (3, 4) liegenden Hälften (4) der Lichtfilter (2) den Lichtstrom (10) mit den aus einem anderen Wellenlängenbereich ausgewählten Wellenlängen durchlassen.

6. Einrichtung nach Anspruch 1 bis 3, dadurch **gekennzeichnet,** dass zumindest ein Lichtfilter (2) im Brillengestell (25) festerfasst ist.

7. Einrichtung zur Korrektion des Gefühlszustandes einer Person nach Anspruch 1 oder 2 oder 3, dadurch **gekennzeichnet,** dass sie mit zwei im Brilllengestell (25) festerfassten Lichtfiltern (2) versehen ist, wobei die an der linken Seite der Trenngrenzen (5) der Filterhälften (3, 4) liegenden Hälften (3) der Lichtfilter (2) den Lichtstrom (11) mit den aus einem Wellenlängenbereich ausgewählten Wellenlängen und die an der rechten Seite der Trenngrenzen (5) der Filterhälften (3, 4) liegenden Hälften (4) der Lichtfilter (2) den Lichtstrom (10) mit den aus einem anderen Wellenlängenbereich ausgewählten Wellenlängen durchlassen.

FIG.1

FIG.2

FIG.3

# INTERNATIONAL SEARCH REPORT

**I. CLASSIFICATION OF SUBJECT MATTER** (if several classification symbols apply, indicate all) ⁶

According to International Patent Classification (IPC) or to both National Classification and IPC

Int.Cl.⁵  A61N5/06

**II. FIELDS SEARCHED**

Minimum Documentation Searched ⁷

| Classification System | Classification Symbols |
|---|---|
| Int.Cl.⁵ | A61N5/06, A61B3/06; C02C7/04, 7/10 |

Documentation Searched other than Minimum Documentation
to the Extent that such Documents are Included in the Fields Searched ⁸

**III. DOCUMENTS CONSIDERED TO BE RELEVANT** ⁹

| Category * | Citation of Document, ¹¹ with indication, where appropriate, of the relevant passages ¹² | Relevant to Claim No. ¹³ |
|---|---|---|
| A | SU,A1, 327927 (Ju. P. Golovko et al.) 22 March 1972 (22.03.72),the drawings | 1-7 |
| A | SU,A1, 179416 (E. B. Rabkin), 05 April 1966 (05.04.66), the claims | 1-3 |
| a | US,A, 3659614 (BERNARD JANKELSON, MEDICAL-DENTAL BUILDING), 02 May 1972 (02.05.72) claim 1 | 1-3 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 23 May 1990 (23.05.90) | 7 June 1990 (07.06.90) |

| International Searching Authority | Signature of Authorized Officer |
|---|---|
| ISA/SU | |

Form PCT/ISA/210 (second sheet) (January 1985)